# EUROPEAN PATENT APPLICATION

(11) **EP 1 250 933 A1**
(43) Date of publication of application: **23.10.2002**
(21) Application number: 01830261.2
(22) Date of filing: 17.04.2001
(51) Int. Cl.: A61K 39/39, A61P 31/00, A61P 35/00, C07K 14/56, C07K 14/565, A61K 39/00, A61K 39/145

(54) **Vaccines including as an adjuvant high dose type I IFN**

(71) Applicant: Istituto Superiore di Sanità, 00161 Roma (IT); The Edward Jenner Institute for Vaccine Research, Newbury, Berkshire RG20 7NN (GB)
(72) Inventor: Belardelli, Filippo, 00152 Roma (IT); Schiavone, Giovanna, 00196 Roma (IT); D'Agostino, Giuseppina, 00155 Roma (IT); Proietti, Enrico, 00147 Roma (IT); Tough, David F., Oxon OX12 9JH (GB); Le Bon, Agnes, Oxford OX1 4TG (GB)
(74) Representative: Bazzichelli, Alfredo

(57) **Abstract**

The present invention relates to the use of type I IFN for the preparation of pharmaceutical composition, in particular vaccine, which include it in a dosage greater or equal to 100.000 U/ml, per dose of vaccine, and to the compositions and in particular vaccine prepared thereby. The present invention further relates to a kit of parts including, type I IFN and a vaccine including an antigen for separate, simultaneous or sequential use in the prevention or treatment of a disease associated with the presence of said antigen.

## Description

### Field of the invention

The present invention relates to vaccines and in particular to sub-unit vaccines.

### Background of the invention

Vaccines are known in the art (1). In general, they include killed or attenuated pathogens and sub-unit vaccines, which are administered with the aim of preventing, ameliorating or treating infectious diseases.

In particular, sub-unit vaccines are vaccines based on antigens derived from components of the pathogen that are considered to be important targets for protection mediated by the host's immune system (1). Although proved to be highly safe, sub-unit vaccines often induce inadequate immune responses due to the fact that the antigen upon which they are based is either poorly immunogenic or non-immunogenic.

Hence, in order to raise immunogenicity, sub-unit vaccines often need to include or be administered together with an adjuvant, i.e. by definition a substance that when administered together with the antigen generates a more effective immune response as compared with the antigen alone (2).

Although many types of adjuvants have been used in animal models and classical examples include oil emulsions, aluminum or calcium salts, saponins and LPS-derived products, currently, aluminum-based mineral salts are the only adjuvants routinely included in the vaccine formulations in humans. Although safe, such salts are weak adjuvants for antibody induction and are not capable of stimulating classical cell-mediated immune responses (3).

Induction of both antibodies and a cell-mediated response are required to provide highly effective defense against invading pathogens with the aim of limiting their spread or eliminating them (4). Vaccines need to provide or induce 2 types of signals in order to elicit a strong, protective immune response. Firstly, vaccines need to deliver the antigen, which triggers antigen-specific receptors on T and B lymphocytes. Secondly, effective vaccines need to induce the expression of co-stimulatory molecules by antigen presenting cells, which then promote a strong response by the antigen-triggered lymphocytes (5,6). This second signal is often provided by factors associated with infection, when using vaccines containing live pathogens, but is generally lacking in sub-unit vaccines, resulting in their poor immunogenicity. The addition of an adjuvant that can contribute this second signal will enhance the effectiveness of the vaccine and, further, may dictate the type of immune response elicited.

The instructive role of these signals to the host's immune system allows the subsequent development of effector mechanisms that characterise the type and potency of the overall immune response to a given infectious agent.

Cytokines represent the major factors involved in the communication between T cells, B cells, macrophages, dendritic cells and other immune cells in the course of an immune response to antigens and infectious agents (7). A number of studies on mouse and human T helper (Th) clones has provided extensive evidence for the existence of different activities exhibited by Th cells (called Th1 and Th2), which was inferred from the profile of cytokine secretion (8). Thus, production of IFN-γ or IL-4 are considered as the typical hallmarks of a Th1 or Th2 response, respectively. The Th1 type of immune response is generally associated with IgG2a production in mice and the development of cellular immunity, whereas the Th2 type of response with IgE production, eosinophils and mast cell production. It is generally thought that induction of a Th1 type of immune response is instrumental for the generation of a protective immune response to viruses and certain bacterial infections. In this regard, it is worth mentioning that clinically available adjuvants such as aluminum-based mineral salts tend to induce a Th2 type of immune response, while the use of some Th1 promoting adjuvants is generally restricted by toxicity or safety issues.

The absence of a highly effective adjuvant in the above sense constitutes a significant obstacle to the successful development of vaccines, particularly those directed against intracellular pathogens, requiring cellular immunity (9).

Accordingly, despite the availability of potentially efficacious recombinant antigens, weakness or absence of responsiveness to vaccination and patient compliance still remains the major concerns for prophylactic or therapeutic sub-unit vaccines. The weak immunogenicity of sub-unit vaccines makes it necessary for these vaccines to be given multiple times in order to elicit a satisfactory response, making lack of patient compliance a significant problem.

Therefore, an adjuvant that improves antigen immunogenicity and promotes consistently strong immune responses, lowering the number of vaccine doses required to induce seroconversion/seroprotection rate even following a single dose, is in fact a long felt need.

In this connection, due to the properties evidenced above, cytokines have been considered in the art as possible adjuvants (10, 11). In particular, among the others, interferon (IFN) has received some attention.

Today, the current view refers to IFNs as a complex family of antiviral proteins secreted by various cells in response to virus infection or other stimuli, which exhibit multiple biologic activities (7). They are classified in 2 major types, depending on the receptor system by which they induce their biological activities (12) :
i) Type I IFNs, which include the IFN-α family of at least 13 functional subtypes of IFN-α, IFN-β and IFN-ω;
ii) Type II IFN, also named IFN-γ.

Originally taken to be simple antiviral substances, type I IFNs have subsequently been shown to exhibit a variety of biological effects, including antitumor activities in experimental models as well as in patients (7). Early studies had reported several effects of type I IFN on the immune response in vitro as well as in vivo. However, some of these studies were viewed with some scepticism since the IFN preparations were in many cases still impure (reviewed in ref. 7). For a long time, it has generally been assumed that the effects of type I IFN on the immune system could not be comparable, in terms of importance, to those exhibited by type II IFN (13), considered as the primary mediator of a protective cell mediated immune response, consistent with its original definition of "immune IFN".

Type I IFNs have also been shown to exert potent inhibitory effects on antibody production (14) and T cell proliferation (15) *in vitro,* raising the question of whether these cytokines would act in a stimulatory or inhibitory manner *in vivo.* It is worth mentioning that several authors have recently emphasized the possible immunosuppressive effects of type I IFN (16 and references therein enclosed). This concept has even led to clinical applications in HIV-1 infected patients based on the rationale of neutralizing endogenous IFN considered as the putative immunosuppressive factor involved in disease progression (17,18). On the other hand, an ensemble of data obtained in different model systems have recently pointed out the importance of type I IFN in the induction of a Th1 type of immune response (8) and in supporting the proliferation, functional activity and survival of certain T cell subsets (13, 19).

Type I interferons are currently the most used cytokines in the clinical practice. In particular, IFN-α is used worldwide in over 40 countries for the treatment of some viral diseases (especially Hepatitis C) and various types of human cancer, including some hematological malignancies (hairy cell leukemia, chronic myeloid leukemia, some B and T cell lymphomas) and certain solid tumors, such as melanoma, renal carcinoma and Kaposi's sarcoma. In contrast, IFN-γ has met poor cases of clinical applications, mostly due to toxicity. Over the last few years, several studies have provided evidence that the biologic effects exerted by type I and type II IFNs can substantially differ in terms of type of activity in different experimental models (reviewed in ref. 7). In some cases, such as melanoma and multiple sclerosis, the clinical use of IFN-γ has led to opposite effects with respect to those achieved with type I IFN.

In spite of its wide clinical use, type I IFN is not yet used as a vaccine adjuvant. This is due to the fact that the state of the art on the role and importance of type I IFN in the regulation of the immune response had remained somehow confusing and controversial.

A relevant use of IFNs *in vivo* as adjuvants in vaccines has been shown only for type II IFN (i.e., IFN-γ). In particular in EP 0241725 a vaccine is described containing a crude protein extract, derived from blood cells of mice infected with the virulent YM line of Plasmodium yoelii, which includes IFN-γ as an adjuvant. The amount of IFN-γ included in the vaccine is indicated in the range of 1.000 to 10.000 U per dose, wherein the amount of IFN-γ producing the adjuvant effect is indicated in 100 to 50.000 U. The dosage used is 5.000 U, even if doses lower than 200 units have been indicated also as effective.

No use of type I IFN as an adjuvant has been properly described, although in WO/8704076 it is disclosed that the possibility of using IFN-α for indirectly potentiating immune response was raised by studies of infection of calves with infectious bovine rhinotracheitis (IBR) virus.

In particular, in WO/8704076 IFN α is administered for this purpose preferably by oral route in a dosage not greater than about 5 IU/lb of body weight per day, with a preferred dosage of 1 IU/lb of body weight. In this connection, administration is carried out in a single dose either simultaneously with the administration of the vaccine or within about one day before or after the vaccine administration. As an alternative WO/8704076 indicates an administration of IFN-α in several doses, for example a dose on two or more of the days in the period consisting of the day before vaccine administration, the day of vaccine administration, and the day after the administration.

### Summary of the invention

The object of the present invention is to provide a safe and highly effective adjuvant capable of significantly improving the immunogenicity of vaccines, in particular killed pathogens or, more specifically, sub-unit vaccines, if included in the relevant composition or administered together with said vaccine as an adjuvant.

Such an object is achieved according to the invention in a first aspect by the use of type I IFN for the preparation of a vaccine including said type I IFN as an adjuvant in a dosage greater than or equal to 100.000 IU per dose of vaccine together with an antigen by the vaccine produced thereby and by a pharmaceutical composition comprising said vaccine together with a pharmaceutically acceptable carrier vehicle or auxiliary agent.

A first advantage of the invention is that the vaccine prepared thereby is able to improve the immune response, characterized by long-term antibody production and immunological memory, as determined by the extent of total antibody production and, more specifically, by the induction of a Th-1 type of immune response (see below).

A second advantage of the invention is in that the vaccine including type I IFN as an adjuvant in the above dosage allows induction of a specific Th-1 type protective humoral and cell-mediated immune responses even with a single vaccine administration.

A third and main advantage of the vaccine above is also the high efficacy in rapidly inducing immune correlates of protection, including induction of a Th-1 type of immune response, in the absence of toxicity, in particular in the absence of toxicity/safety concerns typical of the currently available adjuvants known to promote a Th-1 type of response in animals (for instance: CFA or IFA).

Type I IFN can be any interferon that belongs to this family provided that it is included in the above dosage. In this connection, the most effective dosage in humans is in the range of 1x10⁶ - 6x10⁶ IU.

In a preferred embodiment are used: natural IFN-α (a mixture of different IFN-α subtypes or individual IFN-α subtypes) from stimulated leukocytes of healthy donors or lymphoblastoid IFN-α from Namalwa cells, a synthetic type I IFN, such as *consensus* IFN (CIFN) and IFN-β or recombinant IFN-α subtypes, such as IFN-αa and IFN-αb, or IFN-ω, or new IFN molecules generated by the DNA shuffling method, provided that they are used in the above mentioned dosages indicated per vaccine dose.

Pegylated type I IFN subtypes (20) may be used, with the advantage of a higher *in vivo* half life of IFN after injection, in principle beneficial for achieving a more pronounced and rapid immune response.

Fusion hybrid proteins represented by recombinant type I IFNs fused with monoclonal antibodies capable of targeting dendritic cells might be especially effective as adjuvant to be included in the vaccine formulation.

The vaccine of the invention can include one or even more antigens from an infectious agent or other sources.

This includes purified or partially-purified preparations of protein, peptide, carbohydrate or lipid antigens, and/or antigens associated with whole cells, particularly dendritic cells that have been mixed with the antigen. On the whole, any pathogen can be considered as a possible immunogen to be associated with type I IFN as adjuvant, and can be easily identified by a person skilled in the art.

The amount of antigen(s) present in each vaccine dose is selected as an amount capable of inducing a protective immune response in vaccinated subjects. This amount will depend on the specific antigen and the possible presence of other typical adjuvants, and can be identified by a person skilled in the art. In general, each dose will contain 1-1000 µg of antigen, preferentially 10-200 µg.

Further components can be also present advantageously in the vaccine of the invention. In particular, in a preferred embodiment further adjuvants, and in particular aluminum salts, are included in the vaccine composition.

With regard to form, the vaccine of the invention can be in any form known in the art to be suitable for administering an antigen in association with the adjuvant. Preferably, the vaccine of the invention is formulated for delivering both the antigen and the adjuvant simultaneously to the site of administration.

In some cases, the vaccine can be injected subcutaneously or intramuscularly on the account of the expected effect and ease of use. Intradermal injection can effectively be performed for some vaccines and other delivery systems suitable for recruiting a relevant number of dendritic cells to the injection site could be considered.

Intranasal and oral administration should also be included especially for those infectious agents transmitted through these routes of infection.

For some viral infections, such as influenza virus, intranasal administration of the vaccine can represent a valuable choice, which may result in the induction of a potent protective immunity by using a very practical modality of vaccine delivery.

A person skilled in the art can determine in this connection the most adapt formulation in function of the antigen the vaccination is directed to counteract.

The vaccine of the invention can be formulated in conventional manner, as a pharmaceutical composition comprising sterile physiologically compatible carriers such as saline solution, excipients, other adjuvants (if any), preservatives, stabilizers.

The vaccine can be in a liquid or in lyophilized form, for dissolution in a sterile carrier prior to use. The presence of alum or liposome-like particles in the vaccine formulation are also contemplated, as useful for allowing a slow release of both IFN and the antigens(s). Other strategies for allowing a low release of the IFN-adjuvanted vaccines can be easily identified by those skilled in the art (21) and are included in the scope of this invention.

The pharmaceutically acceptable carrier vehicle or auxiliary agent can be easily identified accordingly for each formulation by a person skilled in the art.

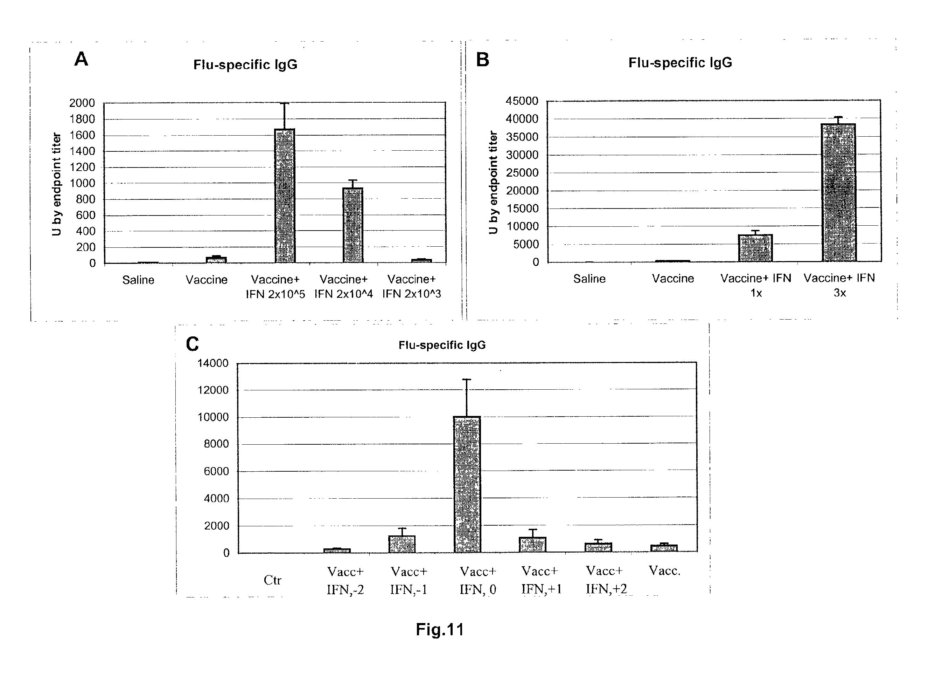

In this connection it is included as an object of the invention also a process for the preparation go the vaccine of the invention comprising the step of:
- formulating together an antigen and type I IFN, said type IFN being in a dosage greater than or equal to 100.000 IU per dose of vaccine.
   The above vaccine can be used in both prophylactic and therapeutic setting for the treatment of infectious diseases and cancer. In particular, the vaccine formulation of the present invention can be used for preventing viral and bacterial diseases (i.e., prophylactic vaccines) as well as for the treatment of severe chronic infection diseases (i.e., therapeutic vaccines). Moreover, the vaccine formulation can be extended for the prevention and treatment of cancer when suitable antigens are used.
   This can be achieved by using antigens against infectious agents associated with human malignancies (EBV, HPV and *E. pilori),* or well defined tumor associated antigens such as those characterized in human melanoma (MAGE antigens, thyrosinase gp100, MART) as well as in other human tumors.
   In particular, vaccine formulation of the invention is particularly suitable for vaccination of the so-called low- or non-responder subjects, such as immuno-compromised subjects like maintenance hemodialysis and transplanted patients. In general, the vaccine of the present invention is advantageously suitable in vaccination of individuals at high risk of infection in any situation for which an earlier seroconversion/seroprotection is desirable.
   These characteristics are in particular referred to vaccination against HBV.
   As an additional example, the described vaccine formulation can be particularly valuable for inducing protection against influenza virus in elderly individuals poorly responsive to standard vaccination.
   For the HBV vaccine as well as for other viral vaccines, the s.c. or intramuscolar route of injection can be preferable, while in other cases the intranasal administration can exhibit advantages in terms or efficacy and/or patient compliance, especially for agents capable of infecting the host through the respiratory system.
   According to a second aspect, the adjuvant effect of type I IFN, according to the invention, is also obtainable by administering type I IFN and the sub-unit vaccine separately.
   In order to be effective, administration should be carried out by modalities allowing the simultaneous presence of the active agents in the same site. In fact, optimal effects are achieved in animals when type I IFN is co-injected together with the vaccine at doses in the range of 100,000-200,000 IU or even at higher doses (1x10⁶ - 2x10⁶ IU).
   Much lower effects are observed when IFN is given alone on day -1 or +1 with respect to vaccine administration (Fig. 11C).
   Accordingly, the present invention includes also a kit of parts comprising
- a composition including type I IFN and a pharmaceutically acceptable carrier;
- a vaccine composition including an antigen or a combination of two or more antigens (defined proteins or peptides) or killed or inactivated pathogens,
   for separate, simultaneous or sequential use in the treatment of a disease associated with the presence of said antigen(s).

Formulation, form and route of administration of the composition of the kit of the invention are the same described above.

The invention will be better described with the help of the annexed figures.

### Description of the figures

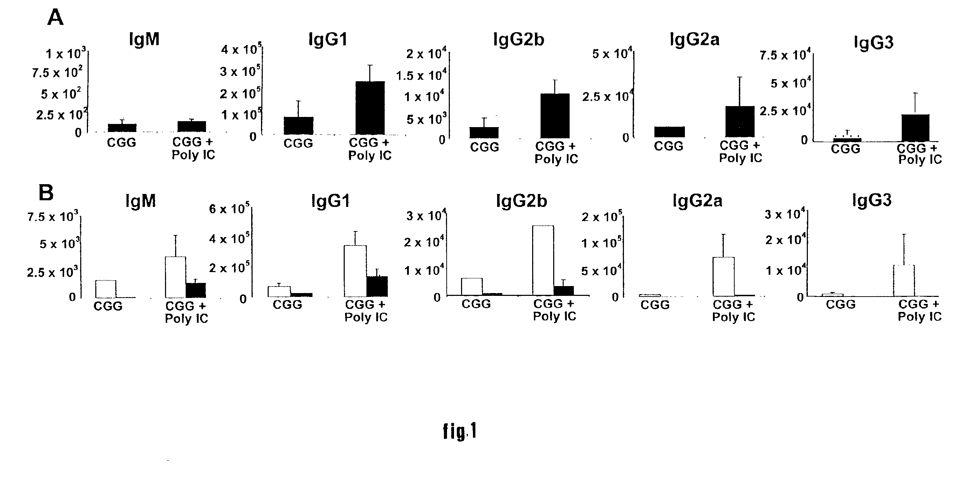

Figure 1 shows the effect of poly(IC) on the primary antibody response to chicken gamma globulin (CGG) *in vivo.*

Panel A shows endpoint titres of CGG-specific antibodies detected in the sera of B6 mice injected with CGG alone or CGG + poly IC, as indicated on the x-axis of the diagram. Each single diagram is labeled with the subclass of the antibody to which the measured endpoint titre refers (IgM, IgG1, IgG2b, IgG2a and IgG3).

Antibody responses are expressed as the mean ± SD of endpoint titres.

Panel B shows the antibody response obtained in wild type mice of the 129sv strain (white bars) or type I IFN receptor KO (type I IFNR KO) 129sv mice (black bars) injected with CGG alone or CGG + poly IC, as indicated on the x axis of the diagram. Each single diagram is labeled with the subclass of the antibody to which the measured endpoint titre refers (IgM, IgG1, IgG2b, IgG2a and IgG3).

Antibody responses are expressed as the mean ± SD of endpoint titres.

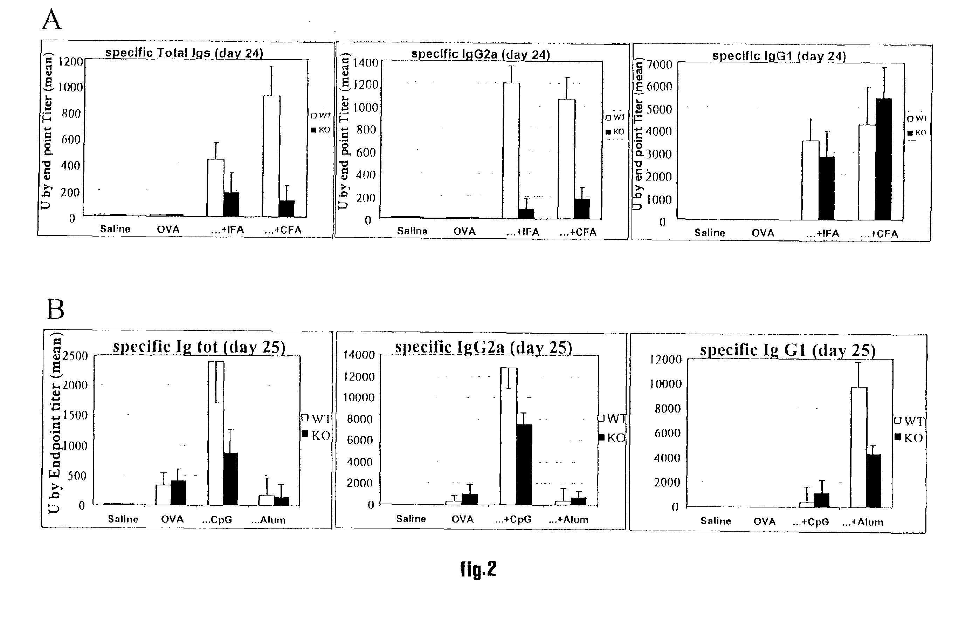

Figure 2 shows antibody response in mice immunized with ovalbumin (OVA) +adjuvants.

Panel A shows specific antibody levels detected in wild type (white bars) or type I IFNR KO C3H/HeJ (gray bars) mice, treated with saline, OVA, OVA+IFA and OVA+CFA as indicated along the x axis of the diagram.

Endpoint antibody titres present in the sera of mice 24 days after immunization, measured by a standard ELISA assay for OVA-specific total Igs, or Ig subclasses IgG2a, and IgG1, are reported on diagram I, diagram II and diagram III respectively. Values are expressed as mean of endpoint dilution titers of three individual sera tested in duplicate.

Panel B shows specific antibody levels in wild type (white bars) or type I IFNR KO C3H/HeJ (gray bars) mice treated with saline, ovalbumin (OVA), OVA+CpG and OVA+Alum as indicated along the x axis of the diagram.

Endpoint antibody titres present in the sera of mice 25 days after immunization, measured by a standard ELISA assay for OVA-specific total Igs or Ig subclasses IgG2a and IgG1, are reported on diagram I, diagram II and diagram III respectively. Values are expressed as mean of endpoint dilution titers of three individual sera tested in duplicate.

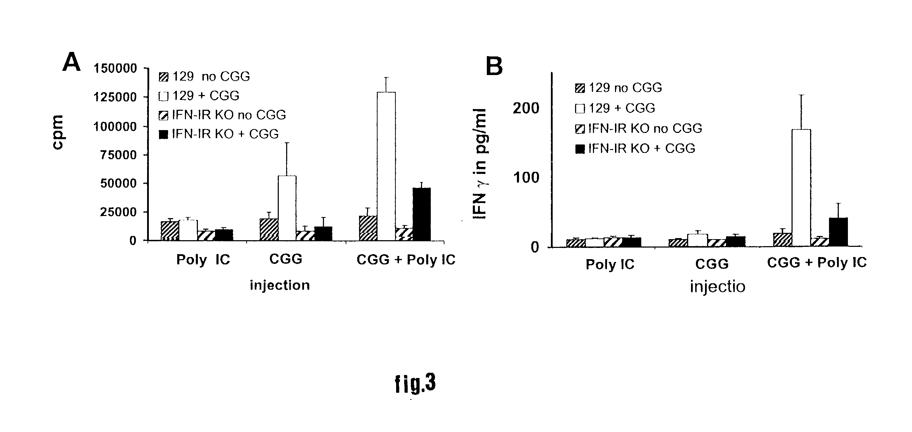

Figure 3 shows the role of type I IFN in Poly IC enhancement of the T cell response to CGG *in vivo.*

Panel A shows the *in vitro* proliferative response to CGG of T cells from 129 or type I IFNR KO mice primed by injection of poly IC, CGG or CGG + poly IC as indicated along the x axis of the diagram.

White and narrow striped bars indicate proliferation by cell suspensions from the draining lymph nodes (DrLNs) of 129 mice cultured in the presence (white) or absence (narrow striped) of CGG.

Black and large striped bars indicate proliferation by cell suspensions from the DrLNs of type I IFNR KO mice cultured in the presence (black) or absence (large striped) of CGG.

Panel B shows IFN-γ secretion by CD4⁺ T cells from 129 or type I IFNR KO mice primed by injection of poly IC, CGG or CGG + poly IC as indicated along the x axis of the diagram.

White and narrow striped bars indicate IFN-γ secretion by CD4⁺ T cells purified from the DrLNs of immunized 129 mice cultured together with T depleted spleen cells from non-immunized syngenic mice cultured in the presence (white) or absence (narrow striped) of CGG.

Black and large striped bars indicate IFN-γ secretion by CD4⁺ T cells purified from the DrLNs of immunized type I IFNR KO mice cultured together with T depleted spleen cells from non-immunized syngenic mice cultured in the presence (black) or absence (large striped) of CGG.

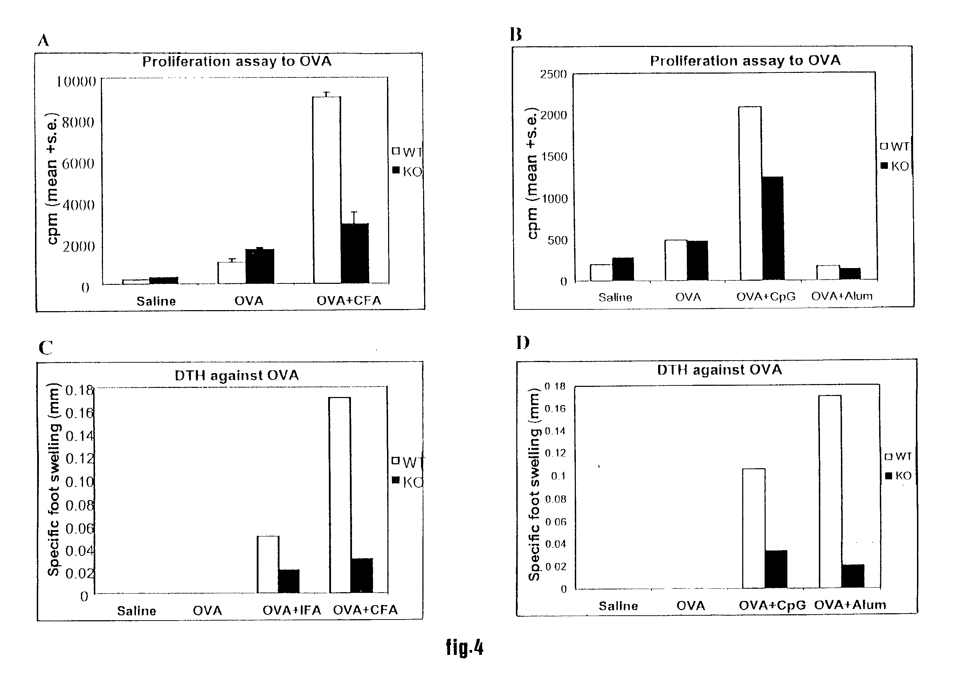

Figure 4 shows the role of endogenous type I IFN in priming T cells for *in vitro* proliferation and in the DTH response in mice immunized with OVA+adjuvants.

Panel A shows specific ³H Thymidine uptake by T cells from normal (white bars) or type I IFNR KO C3H/HeJ (gray bars) mice treated with saline, ovalbumin and CFA as indicated along the x axis of the diagram.

Panel B shows specific ³H Thymidine uptake by T cells from normal (white bars) or type I IFNR KO C3H/HeJ (gray bars) mice treated with saline, ovalbumin and ovalbumin plus CpG or Alum, as indicated along the x axis of the diagram.

Panel C shows the specific DTH response in wild type (white bars) or type I IFN receptor KO C3H/HeJ (gray bars) mice treated with saline, ovalbumin and ovalbumin plus IFA or CFA, as indicated along the x axis of the diagram.

Panel D shows the specific DTH response in wild type (white bars) or type I IFN receptor KO C3H/HeJ (gray bars) mice treated with saline, ovalbumin and ovalbumin plus CpG or Alum, as indicated along the x axis.

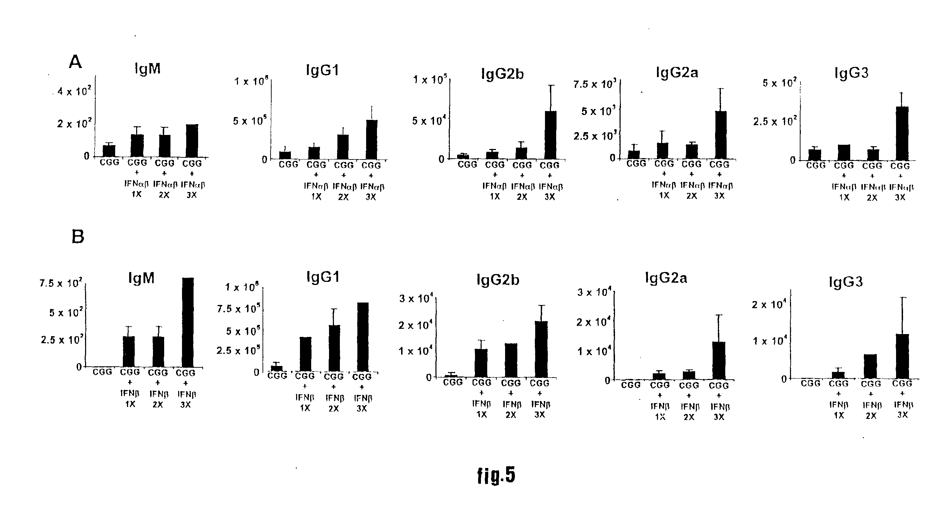

Figure 5 shows enhancement of the primary antibody response to CGG by injection of type I IFN. All mice received a single injection of CGG. Those also receiving soluble IFN-α/β or IFN-β were injected with the respective IFN either only at the time of immunization (1X), or given additional injections of IFN 1 day later (2X) or 1 and 2 days later (3X).

Panel A shows the antibody response in B6 mice immunized with CGG alone or immunized with CGG and treated with IFN-α/β, as indicated along the x-axis of the diagram.

Each single diagram is labeled with the subclass of the antibody to which the measured endpoint titre refers (IgM, IgG1, IgG2b, IgG2a and IgG3). Antibody responses are expressed as the mean ± SD of endpoint titres.

Panel B shows the antibody response in B6 mice immunized with CGG alone (black bars) or immunized with CGG and treated with IFN-α/β (white bars).

Each single diagram is labeled with the subclass of the antibody to which the measured antibody concentration refers (IgM, IgG1, IgG2b, IgG2a and IgG3).

Data show the concentration of CGG-specific antibody detected in mice receiving CGG alone or CGG + 3 injections of IFN-α/β 10 days after immunization. Approximate quantities of CGG-specific antibodies were determined by comparison with isotype standards (3 mice per group).

Panel C shows the frequency of cells secreting CGG-specific antibodies in DrLNs of B6 mice immunized with CGG alone (white bars) or immunized with CGG and treated with IFN-α/β (black bars), compared to an non-immunized control (gray bars).

Data are expressed as the number of cells secreting CGG-specific antibodies of particular isotypes per 5 X 10⁵ total LN cells detected at day 10 after immunization in mice receiving CGG alone or CGG + 3 injections of IFN-α/β.

Panel D shows the antibody response in B6 mice immunized with CGG alone or immunized with CGG and treated with IFN-β. Each single diagram is labeled with the subclass of the antibody to which the measured endpoint titre refers (IgM, IgG1 IgG2b, IgG2a and IgG3). Antibody responses are expressed as the mean ± SD of endpoint titres.

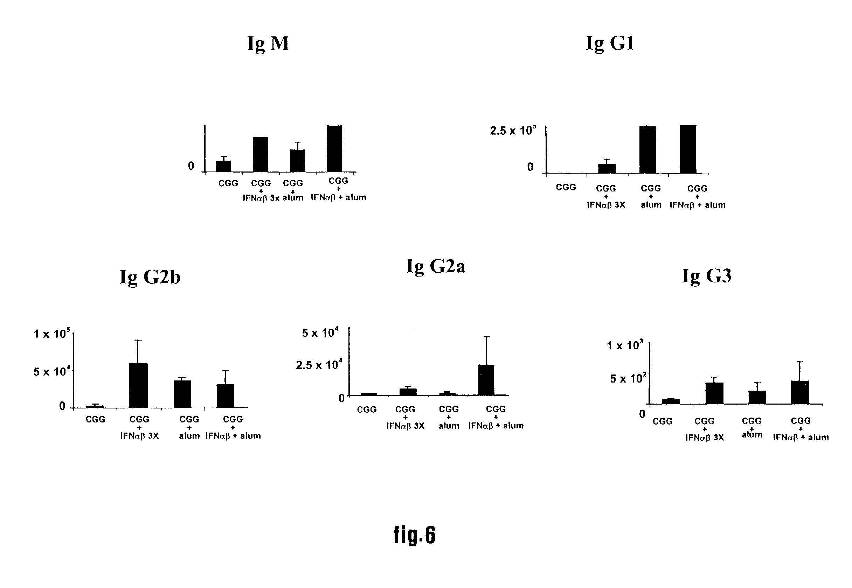

Figure 6 shows enhancement of primary antibody response to CGG by IFN-α/β+ alum.

Data (black bars) show CGG-specific antibodies detected by ELISA 10 days after immunization of B6 mice by giving a single subcutaneous injection of CGG alone or in combination with soluble IFN-α/β, alum, or IFN-α/β+ alum, as indicated along the x axis of the diagrams. Symbol 3x indicates additional injections of soluble IFN-α/β 1 day and 2 days after the immunization.

Each single diagram is labeled with the subclass of the antibody to which the measured endpoint titre refers (IgM, IgG1, IgG2b, IgG2a and IgG3).

Data represent the endpoint titres ± SD (3 mice per group).

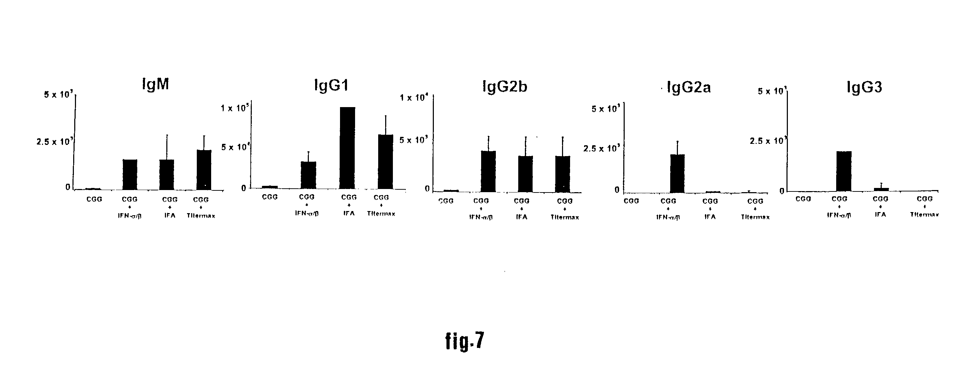

Figure 7 shows a comparison of antibody responses enhanced by type I IFN and oil-based adjuvants.

Data (black bars) show CGG-specific antibodies detected by ELISA 10 days after immunization of B6 mice by subcutaneous injection of CGG, CGG + IFN-α/β (IFN-α/β given together with CGG on day 0 and then alone on day 1 and 2), CGG emulsified in IFA (IFA given together with CGG on day 0 and then alone on day 1 and 2) or CGG emulsified in TiterMax (TiterMax given together with CGG on day 0 and then alone on day 1 and 2) as indicated along the x axis of the diagrams.

Results are expressed as the mean ± SD of endpoint titres (3 mice per group). Each single diagram is labeled with the subclass of the antibody to which the measured endpoint titre refers (IgM, IgG1, IgG2b, IgG2a and IgG3).

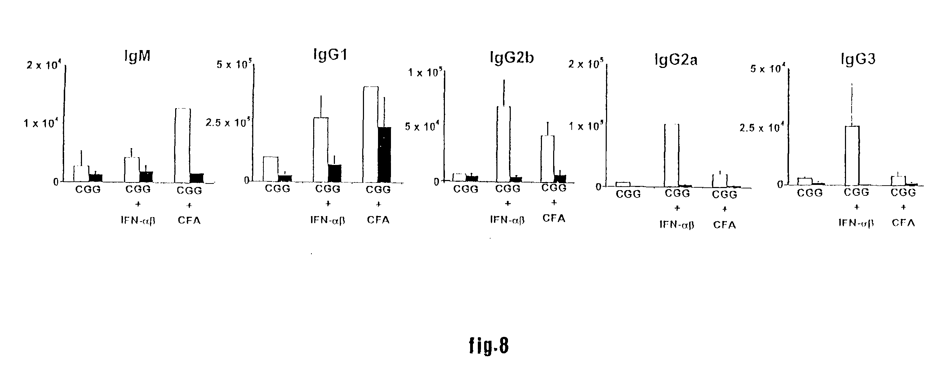

Figure 8 shows that soluble IFN-α/β enhances antibody responses to a similar extent as CFA, the adjuvanticity of which is dependent on endogenous type I IFN.

Antibody responses were compared in WT 129 (white bars) or IFN-IR KO (black bars) mice after immunization by subcutaneous injection of CGG alone, CGG + IFN-α/β (two more IFN-α/β injections given on day 1 and 2 post immunization) or CGG + CFA, as indicated along the x-axis of the diagrams. CGG-specific antibodies were detected by ELISA 10 days after immunization. Results are expressed as the mean ± SD of endpoint titres (3 mice per group). Each single diagram is labeled with the subclass of the antibody to which the measured endpoint titre refers (IgM, IgG1, IgG2b, IgG2a and IgG3).

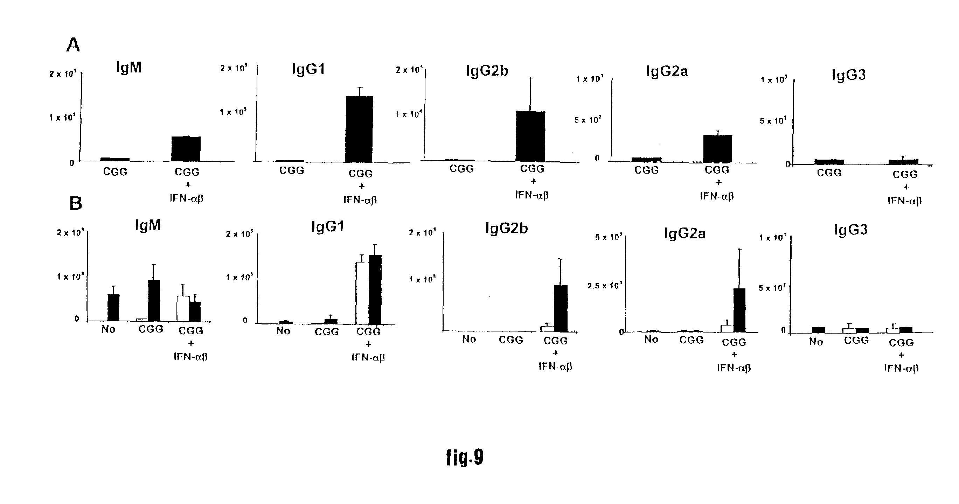

Figure 9 shows type I IFN stimulation of long-term antibody production and immunological memory.

Panel A shows endpoint antibody titres in B6 mice immunized 6 months earlier by injection of CGG alone or CGG + IFN-α/β.

Data (black bars) show CGG-specific antibodies detected by ELISA 6 months after immunization of B6 mice by subcutaneous injection of CGG alone or CGG + IFN-α/β (two more IFN-α/β injections given on day 1 and 2 post immunization).

Results are expressed as the mean ± SD of endpoint titres (3 mice per group). Each single diagram is labeled with the subclass of the antibody to which the measured endpoint titre refers (IgM, IgG1, IgG2b, IgG2a and IgG3).

Panel B shows specific antibody response 6 days after challenge with CGG alone in naïve mice (No) and in mice immunized 6 months before with CGG alone or CGG + IFN-α/β as in panel A.

Results represent antibody endpoint titres before (white bars) and 6 days after challenge (black bars) expressed as the mean ± SD of endpoint titres (3 mice per group). Each single diagram is labeled with the subclass of the antibody to which the measured endpoint titre refers (IgM, IgG1, IgG2b, IgG2a and IgG3).

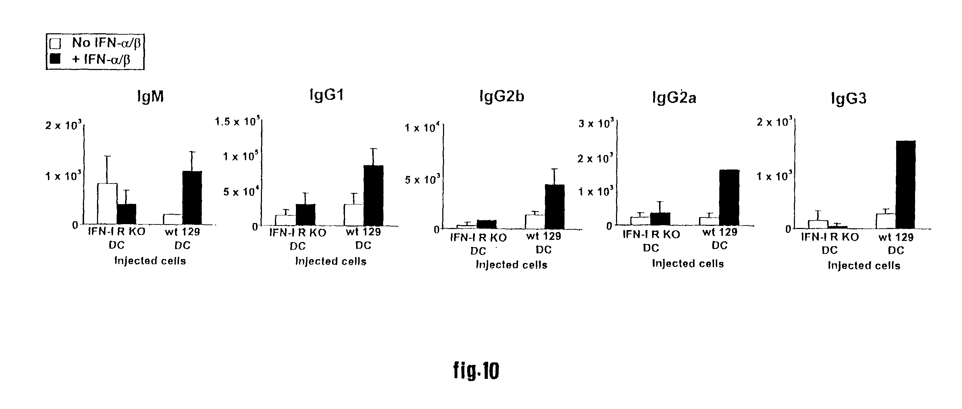

Figure 10 shows that DC responsiveness to type I IFN is sufficient for enhancement of antibody production and isotype switching by type I IFN.

Data show CGG-specific antibodies detected by a standard ELISA 10 days after immunization. Splenic DC were purified from 129 mice (wt) or from type I IFNR KO mice (as indicated along the x axis of the diagrams), incubated briefly with CGG and injected with (black bars) or without (white bars) IFN-α/β sc into type I IFNR KO mice (in the latter case two more IFN-α/β injections given on day 1 and 2 post immunization).

Results are expressed as the mean ± SD of endpoint titres (3 mice per group). Each single diagram is labeled with the subclass of the antibody to which the measured endpoint titre refers (IgM, IgG1, IgG2b, IgG2a and IgG3).

Figure 11 Adjuvant activity of type I IFN on antibody response of mice immunized with influenza vaccine.

The standard immunization schedule was as follows: 7-8 week-old C57BL/6 mice were injected i.m. with 0.2 ml of a preparation containing 15 µg of purified flu vaccine and 2x10⁵ U of murine type I IFN, vaccine alone or saline as controls. 14 day later, mice were bled and sera tested by a standard ELISA to measure flu-specific antibody level. Values represent the mean of 5 individual sera ± SD.

Panel A shows a dose/response experiment in which mice were treated i.m. with different preparations containing vaccine mixed with log₁₀ dilutions of type I IFN (2x10⁵, 2x10⁴or 2x10³ Units) or vaccine alone or saline, as a negative control. Antibody titer was measured 7 days after immunization.

Panel B shows the adjuvant effect on antibody response in mice treated with vaccine and a single or a repeated dose of type I IFN. Mice were treated i.m. with flu vaccine mixed with IFN or flu vaccine mixed with IFN, followed by a further IFN injection in the same site for two days after the first inoculum. Flu vaccine alone or saline were used as controls.

Panel C shows the adjuvant effect type I IFN given simultaneously or at different times before or after flu vaccine administration. Mice were injected i.m. with IFN 2 days or 1 day before or after vaccine administration or at the same time of vaccine, at the same site. Flu vaccine alone or saline were used as controls.

### Figure 12

Panel A shows antibody response of mice given an intranasal administration of type I IFN and influenza vaccine.

7-8 week-old C57BL/6 mice were anaesthetized and instilled intranasally (i.n.) with a drop (50 µl) of flu vaccine (15 µg) preparation containing 5x10⁴ Units type I IFN. 14 days later, treatments were repeated and after 7 additional days blood samples were taken and assessed in a standard ELISA for the presence of different flu-specific antibody subclasses. Flu vaccine alone or saline were used as controls. Results are expressed as the mean ± SD. of endpoint titres of 5 mice per group.

Panel B shows the protective effect of IFN adjuvanted vaccine in mice inoculated with live influenza virus.

7-8 week-old C57BL/6 mice were vaccinated i.m. with 0.2 ml of a solution containing flu vaccine (15 µg) alone or in association with type I IFN (2x10⁵ U) or with saline as control. Vaccines were administered on days 0 and 14. Fifty days after the vaccination onset, mice were instilled intranasally with a drop (50 µl) of 10 LD50 of live flu virus (A/Beijing/262/95(A/H1N1). All mice were weighted daily thereafter. Results are expressed as the mean weight of 5 mice per group. The number of surviving mice out of total mice in each group is indicated.

### Detailed Description of the Invention

Type I IFN suitable in the invention is any IFN that belongs to this family, both as single recombinant molecule or as a pool of natural or recombinant molecules, or the *consensus* IFN (CIFN).

For vaccines administered to humans, human type I IFN are the preferred adjuvants. The adjuvant can be a recombinant IFN-α or IFN-β or IFN-ω, the natural IFN-α(a mixture of different IFN-α subtypes or individual IFN-α subtypes) from stimulated leukocytes of healthy donors or lymphoblastoid IFN-α from Namalwa cells, or the CIFN, or new IFN molecules produced *in vitro* by DNA shuffling method and endowed with biological activity.

For veterinary use, type I IFN consist of those naturally found in or closely related to the species for which vaccines are prepared; again, these type I IFN may be recombinant or naturally produced from appropriate animal cells. We noted that all these types of interferon have approximately the same adjuvant activity.

The amount of interferon required to achieve an optimal adjuvant activity depends on the type of the antigen (i.e. its immunogenicity), but typically should be more than 100.000 IU per dose of vaccine. In mice, we found that optimal effects are obtained by injecting high doses of type I IFN (2x10⁵ - 10⁶ IU). Optimal dosage in humans is expected to be in the range of 10⁶-6x10⁶ IU per vaccine dose. Pegylated type I IFN subtypes (20) have the advantage of allowing a higher in vivo half life of IFN after injection, which could be beneficial for achieving a more pronounced and rapid immune response.

Fusion hybrid proteins represented by recombinant type I IFNs fused with monoclonal antibodies capable of targeting dendritic cells (for instance anti-DEC-205 or anti-CD11c antibodies) might be especially effective as adjuvant to be included in the vaccine formulation.

In addition or in alternative to the administration of IFN protein, the adjuvant can also be given as nucleic acid sequence, provided with appropriate regulatory regions for its correct expression, encoding one or more members of type I IFN (i.e. a plasmid containing a type I IFN encoding gene, under the control of appropriate promoter and transcription termination signal sequence, for expression in eukaryotic cell system).

The adjuvant activity refers to any portion of interferon capable of enhancing the antigen specific immune response.

Vaccine composition should preferably contain both antigen and adjuvant, blended in the same vial in physiologically compatible carriers (e.g. sterile saline solution, buffered at physiological pH).

In this connection, the vaccine of the invention can include one or even more antigens from an infectious agent or other sources as well as killed or attenuated pathogens associated with an effective amount of biologically active type I IFN. The vaccine can also include whole cells, and, in particular, autologous dendritic cells.

Antigens for such a formulation can be any type of natural or recombinant purified antigen, which may include protein, peptide, lipid or carbohydrate antigens, derived from intracellular or extracellular pathogen, including viruses, bacteria, protozoa, and fungi, as well as cellular antigens associated with tumors.

The amount of antigen(s) present in each vaccine dose is selected as an amount capable of inducing an immunoprotective response in vaccinated subjects. This amount will depend on the specific antigen and the possible presence of other typical adjuvants. In general, each dose will contain 1-1000 µg of antigen, preferentially 10-200 µg.

Antigens can be any type of natural or recombinant antigen, or its portion, derived from intracellular or extracellular pathogens, as well as the pathogen itself, including viruses (picornaviruses, caliciviruses, coronaviruses, arenaviruses, parvoviruses, togaviruses, flavivirus, coronavirus, rhabdoviruses, filoviruses, ortomixoviruses, paramixoviruses, buniaviruses, retroviruses, papovaviruses, adenoviruses, herpesviruses, poxviruses, hepadnaviruses), bacteria (Streptococci, Staphylococci, Neisseria, Spirochetes, Clostridia, Corynebacteria, Listeria, Erysipelothrix, Anthrax, Mycobacteria, Enterobacteriaceae, Vibrio, Campylobacter, Helicobacter, Haemophilus, Bordetella, Brucella, Francisella, Pasteurella, Yersinia, Chlamydia, Rickettsiae and other non fermentative Gram-negative Bacilli), Mycoplasma and Legionella, protozoa (Sarcodina, Ciliophora, Mastigophora, Sporozoa, Cryptosporodium, Pneumocystis), fungi (Coccidioides, Histoplasma, Blastomycoses, Cryptoccus, Candida, Aspergillus, Mucorales, Zygomyces).

Tumor-associated antigens include melanoma antigens (MART-1, gp100, MAGE antigens) as well as other tumor antigens known in the art (22, 23).

The vaccine of the invention can be formulated in conventional manner, using sterile physiologically compatible carriers such as saline solution, excipients, other adjuvants (if applicable), preservatives, stabilizers. The vaccine can be in a liquid or in lyophilized form, for dissolution in a sterile carrier prior to use.

In another aspect, this invention provides a method of formulating a vaccine, which includes antigens or their portion from a pathogen, in association with an effective amount of biologically active type I IFN acting as an adjuvant, for delivery of both antigen and adjuvant simultaneously to the site of administration. The amount of IFN must be high enough to act locally and for a sufficient time in order to exert its adjuvant effect.

This is an important aspect of vaccine formulation, both to keep antigen and adjuvant mixed together at the same relative concentration following injection, and to expose them contemporaneously to antigen presenting cells, localised at the site of injection, on which antigen and adjuvant exert their functional activity.

In addition to the applications related to the field of prophylactic vaccines, due to the capability of type I IFN of acting as a powerful adjuvant not only with regard to humoral immunity but also to cell-mediated immune responses, this invention is also transferred to the development of therapeutic vaccines for treatment of chronic diseases, such as viral chronic infection and cancer.

In this case, such a new vaccine formulation should contain a tumor-associated antigen or the relevant viral antigen (or the DNA sequence encoding for that antigen), combined with an effective amount of type I IFN to be administered to patients for the treatment of cancer or chronic viral diseases.

Subcutaneous injection of the vaccine is preferable, in the case of the single formulation, because of their simplicity of use. However, any other route of administration may be employed, including intramuscular, intradermal, intranasal and oral routes. For some viral infections, such as influenza virus, intranasal administration of the vaccine can represent a valuable choice, which may result in the induction of a potent protective immunity by using a very practical modality of vaccine delivery.

Notably, a commercially available vaccine, obtained by purification of H1N1 influenza virus circulating in 1995(A/Beijing/262/95(A/H1N1)), proved to be poorly immunogenic when injected in mice. In fact, a consistent number of mice did not develop any significant antibody response even after three vaccine injections. When vaccine was administered i.m. together with type I IFN, 100% of mice seroconverted after a single immunization, and antibody titres increased significantly after a second injection.

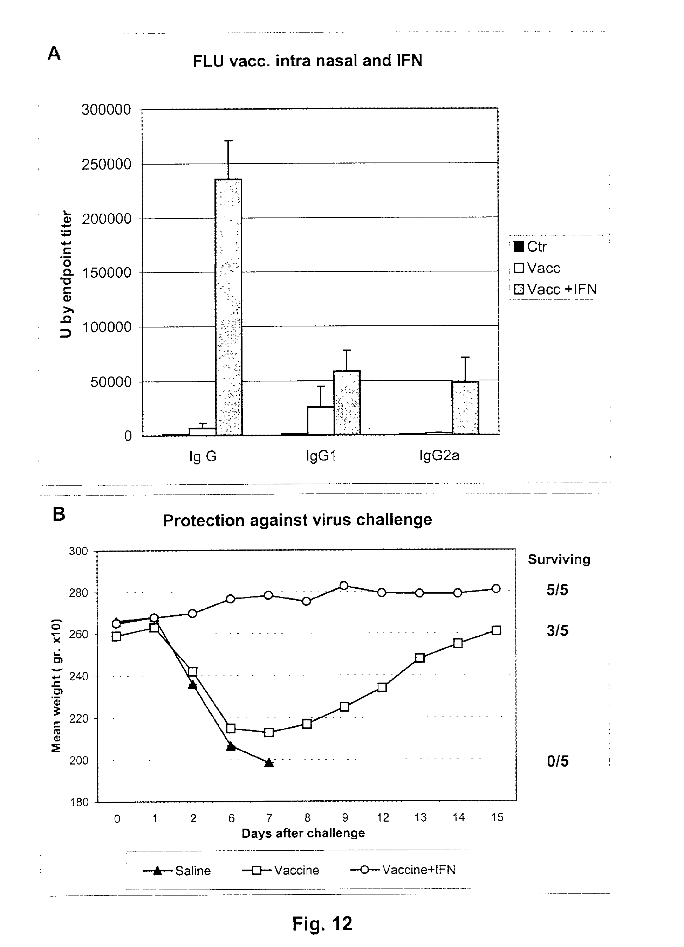

The dose response curve indicated that there was a linear correlation between the IFN dose and antibody titre (Fig. 11). The analysis of influenza specific antibody isotype showed an increase in the IgG2a antibody subclass, a typical marker of the protective Th-1 immune response in mice (not shown). Of interest, mice injected intranasally with 50 µl of a preparation containing the vaccine and type I IFN developed a systemic and mucosal antibody response, while vaccine alone was totally ineffective (Fig. 12a). Notably, antibody response of mice immunised with adjuvanted vaccine was shown to be protective against a virus challenge administered at lethal dose (Fig. 12b).

The amount of interferon required for these methods of administration is similar to that required for subcutaneous route. As inferred, for instance, from the results with the influenza vaccine in mice, one suitable dose of type I IFN can be sufficient to elicit considerable levels of specific immune response. However, the administration of a further dose of adjuvant one or two days following the first IFN-adjuvanted vaccine dose improves the overall magnitude of immune response.

In particular, mixing type I IFN with the relevant antigen and alum prior to injection may be advantageous (Fig. 6), in agreement with a previously reported approach shown to greatly augment the adjuvanticity of IL-12 (24), leading to new formulations capable of promoting a Th-1 type of immune response.

In fact, when pre-adsorbed to alum, a single injection of IFN in mice enhanced the antigen specific antibody response to a similar or greater extent than 3 injections of soluble IFN (Fig. 6). The augmenting effect of alum pre-absorption was most marked with regard to IgG2a production, indicating a Th-1 type preferential response.

Accordingly, a prolonged presence of type I IFN does indeed increase its adjuvant activity. In this regard, pegylated type I IFN may have some advantage because of their high half life after injection (20). Moreover, a vaccine composition suitable for human application, characterised by controlled and prolonged released of both antigen and adjuvant is also contemplated. Such a composition refers to routinely used methods employed to improve functional activity of therapeutic proteins through sustained release formulation (21). These methods make use of formulations in which proteins are encapsulated in microspheres made of biodegradable polymers or liposomes, from which they are slowly released.

If necessary, following the administration of the first vaccine dose, boost doses may be administered to subjects, depending on the immunogenicity of antigen used and the parameter immunization coverage established by specific vaccination programmes.

Alternatively, the adjuvant effect of IFN, even if less pronounced, can be obtained by administrating type I IFN separately from the antigen as a kit of part, but very close to the antigen injection site and at the same time. Subcutaneous injection of vaccine is preferable, also in the kit of part composition, because of their simplicity of use. However, any other route of administration may be employed, including intramuscular, intradermal, intranasal and oral routes. The amount of interferon required for these methods of administration is similar to that required for subcutaneous route.

The present invention is based on the following unexpected major findings:
i) the simultaneous injection of well defined antigens mixed together with a suitable amount of type I IFN in mice results in a powerful induction of a primary antibody response, associated with a typical Th-1 type of immune response (Figs. 5, 6, 9), which is superior to that observed with the use of typically available adjuvants (Fig. 7), without inducing any toxicity;
ii) endogenous type I IFN is the major mediator of the Th-1 promoting immune response induced by adjuvants such as IFA, CFA, CpG, when coinjected with reference antigens (Figs. 1-4, 8) (all these adjuvants pose relevant safety issues to be used in humans) ;
iii) a commercially available influenza vaccine, when administered intramuscularly or intranasally in mice together with suitable amounts of type I IFN, becomes highly immunogenic and protective against virus challenge(Figs. 11, 12).

The importance and the details of these findings are clarified in the examples reported below.

### Examples

### Materials and Methods

### Mice

Mice were purchased from Charles River-UK (Margate, Kent, UK), Charles River-Haly (Calco, Italy) or from the SPF unit at the Institute for Animal Health (Compton, UK). C3H/HeJ mice were purchased from Harlan UK Ltd (Blackthorn, UK). 129 SvEv (129) mice were purchased from the SPF unit at the Institute for Animal Health. 129 background mice deficient for type I IFN receptor function (type I IFNR KO) were originally purchased from B&K Universal (North Humberside, UK) and were maintained and bred in the SPF unit at the Institute for Animal Health.

### Interferons

High titer IFN-α/β x 10⁷ U/mg of protein was prepared in the C243-3 cell line following a method adapted from Tovey et al (25). Briefly, confluent cells were primed by the addition of 10 U/ml of IFN in MEM enriched with 10% FCS and 1mM Sodium Butyrate. After 16 hours of culture at 37° C, C243-3 cells were infected by Newcastle Disease Virus (multiplicity of infection of 1) in MEM + 0.5 % FCS + 5mM theofylline. 18 hours post-infection, culture supernatant was collected and centrifuged at 1500 rpm for 10 min. The supernatant was adjusted to pH 2.0 and kept at 0°C for 6 days, before IFN titration. IFN was assayed by inhibition of the cytopathic effect of vesicular stomatitis virus on L cells in monolayer culture in Falcon microplates. These IFN preparations had the specific activity of 2x10⁶ U/mg of protein after removal by centrifugation of contaminating protein precipitated during the treatment at pH 2.0 and dialysis against PBS. Units in the text are expressed as international mouse reference units. IFN was concentrated and partially purified by ammonium sulfate precipitations and dialysis against PBS. All IFN preparations were further subjected to dialysis for 24 hr at 4°C against 0.01 M percloric acid and then against PBS, before testing them for any possible residual toxicity on a line of L1210 cells resistant to IFN. These partially purified IFN preparations had a titer of at least 2x10⁷ U/mg of protein and were endotoxin-free, as assessed by the *Limulus* amebocyte assay. They proved to be constituted of approximately 75% IFN-β and 25% IFN-α, as evaluated by neutralization assays using mAbs to IFNs, as described in detail elsewhere (26). High titre purified IFN-β (2 x 10⁹ U/mg of protein) was prepared by affinity chromatography on a Sepharose column coupled with rat monoclonal antibodies to IFN-β (27).

### Antigens and adjuvants.

Chicken Gamma Globulin (CGG) and Ovalbumine (OVA) were obtained, respectively, from Stratech Scientific Ltd, Luton, UK and Sigma Chemical Co. Influenza vaccine was a sub-unit X-127 monovalent vaccine, prepared from A/Beijing/262/95 (H1/N1) influenza virus strain and was kindly provided by Chiron Corporation (Emeryville, CA).

Antigens were dissolved in PBS and filter-sterilised. Incomplete (IFA) and Complete Freund Adjuvant (CFA) (Sigma Chemical Co), were each mixed with antigen solution at a 1:1 v/v ratio and emulsified, by using two glass syringes and luer lock connectors, until a stable emulsion was formed. Alum (aluminum hydroxide gel, Sigma Chemical Co) was dissolved in the antigen solution at a ratio 1:20 w/v and the pH was adjusted to 6.5. After 1 h incubation at room temperature, the solution was centrifuged and the pellet resuspended in the previous volume of saline. CpG ODN (CpG) was synthesized by Roche Diagnostic, Milan, Italy. 200 µg of CpG were dissolved in 1 ml, final volume, of a solution containing 200 µg of OVA. The CpG used in this study was made with a phosphorothioate backbone and had the sequence TsGsAsCsTsGsTsGsAsAsCsGsTsTsCsGsAsGsAsTsGsA. Polyinosinic-polycytidylic acid (Poly (I:C) (Sigma Chemical Co) was dissolved in saline at a concentration of 10 mg/ml. Frozen aliquots were thawed just before each experiment and 0.15 mg of poly (I:C) were injected i.p. in a volume of 0.15 ml of saline.

### Protocols of immunizations with CGG

All immunisations were done by sc injection of 100 µg of CGG. CGG was administrated in soluble form (in PBS) either when given alone or when mixed with 100 µg of poly IC (Sigma Chemical Co. Ltd, Dorset, UK), 10⁵ U of IFN-αβ or 10⁵ U of purified IFN-β as indicated. When injected with Titermax (CytRx Corporation, Norcross, GA), IFA (Sigma) or CFA (Sigma), an equal volume of CGG in PBS was emulsified with the adjuvant before injection. In some experiments, type I IFN, Titermax or IFA were administered several times. In all cases, mice were injected sc at the site of the primary injection. When testing for a memory response, mice were bled immediately prior to challenge with CGG to establish pre-challenge antibody levels. The same mice were bled 6 days after CGG challenge.

### Protocols of immunisations with OVA

Except for Poly (I:C), all adjuvants were always injected i.d. in a volume of 50 µl/mouse with or without OVA. OVA concentration was always 10 µg/mouse. Two different immunization protocols were used. To achieve a good antibody and proliferative response, mice were injected at day 0 with OVA+adjuvant and 10 and 17 days later boosted with OVA alone. On the contrary, mice selected for delayed type hypersensitivity (DTH) assay were injected with OVA+adjuvant both at time 0 and at times 10 and 17. All immunization experiments included arms treated with OVA alone and saline as controls. Blood samples were taken from the retro-orbital venous plexus just before the subsequent antigen injection. Sera were collected and stored at -20°C before further assay.

### Protocols of immunizations with influenza vaccine

For intramuscular (i.m.) immunization, mice were injected with a final volume of 200 µl of a solution containing vaccine (15 µg) and saline, or vaccine (15 µg) and IFN (2x10⁵ U), or saline alone. For intranasal (i.n.) immunization, lightly anaesthetized mice were instilled with a drop (50 µl) of a solution containing the same amounts of vaccine and saline, or vaccine and IFN, or saline alone. Booster dose containing identical amounts of vaccine and IFN was applied 14 days after primary immunization.

### Assay of Serum Antibody by ELISA

CGG (5µg/ml in Carbonate Buffer-pH 9.6) was coated overnight at RT in 96-well Flexible Plates (Falcon, Becton Dickinson, Oxford, UK). The plates were blocked with PBS containing 4% powdered milk for 1 hour at 37° C and then washed 3X in PBS-Tween (0.05%). 12-fold serial dilutions of sera in PBS-1% milk were added to the wells for 1 hour at RT. After 3 washes, biotinylated rat anti-mouse antibodies [anti-mouse IgM (R6-60.2), IgG1 (A85-1), IgG2a (R19-15), IgG2b (12-3), IgG3 (R40-82) or IgE (R35-72) (Becton Dickinson)] were added to the wells for 1 hour at RT. After 3 washes, streptavidin-HRP (Becton Dickinson) was added for 1h at RT. OPD tablets (Sigma) were used as peroxidase substrate. The reaction was stopped by addition of 50 µl 3M HCl before the highest dilution of the highest titre serum rose above background. Optical densities were read at 492 nm on a SPECTRAmax (Molecular Devices, Sunnyvale, CA). Results are expressed as reciprocal endpoint titres, which were determined using an automated routine designed on Excel. Briefly, a threshold of positivity for OD values was calculated for each antibody isotype as the average + 3 SD of all dilutions from 3 control mouse sera (sera from either unmanipulated mice or mice treated with IFN-α/β or poly IC alone). The background level was very low at all dilutions (typically about 0.08) and did not vary significantly between experiments. For a given serum sample, the endpoint titre was determined as the first dilution below the threshold of positivity. Since endpoint titres are arbitrary units, the results must be considered inside the same assays and cannot be directly compared between experiments. For this reason, all samples within each experiment were assayed at the same time.

To determine the approximate concentrations of CGG-specific antibodies present in mouse sera, the ELISA was performed in a semi-quantitative way by comparison to mouse Ig standards. CGG-specific antibodies were detected as described above, except that antibodies were revealed using isotype specific polyclonal goat anti-mouse antibodies conjugated to alkaline phosphatase (AP) (all from Southern Biotechnology Associates Inc, Birmingham, AL, USA). To establish standards, plates were coated with unlabelled isotype specific polyclonal goat anti-mouse antibodies (5 µg/ml) (Southern Biotechnology). The plates were blocked as above and then purified mouse antibodies were added at known concentration (mouse Ig Standard Panel from Southern Biotechnology). After washes, the standards were revealed using isotype specific goat anti-mouse antibodies conjugated to AP. p-NPP tablets (Sigma) were used as the AP substrate. The enzymatic reaction was stopped by adding 3M NaOH. OD was read at 405 nm. Using SoftmaxPro (Molecular Devices, Sunnyvale, CA), we established standard curves for each isotype and calculated the amount of CGG specific antibodies.

To measure OVA-specific antibody levels, a standard, direct ELISA assay was performed. Briefly, 96 well flat-bottom microtiter plates (DYNEX Immulon 4MBX) were coated with 100 µl of a 1 µg/ml (for total IgG detection) or 4 µg/ml (for IgG2a and IgG1 detection) solution of ovalbumine (Sigma Chemical Co, St.Louis, MO) diluted in NaHCO₃ buffer, pH 9.6 (coating buffer). After overnight incubation at 4°C, plates were washed three times with PBS + 0.01% Tween 20 (washing solution) and blocked with PBS containing 5% bovine serum albumin (BSA) (Sigma Chemical Co, St. Louis, MO) for 2 hr at room temperature. 100 µl of previously diluted (to a 1:2 ratio) serum samples were then added to each well and incubated for 1h and 30 min at 37°C. After washing, plates were incubated 1h at 37°C with 100 µl of a peroxidase-conjugated secondary antibody. The following secondary antibodies were used: anti total mouse IgG (Sigma Chemical Co, St. Louis, MO), diluted 1:1000 in PBS containing 5% BSA; anti mouse IgG2a (Cappel Research Products, Durham, NC), diluted 1:200; anti mouse IgG1 (Cappel Research Products, Durham, NC), diluted 1:400. At the end of the incubation, plates were washed three times and 100 µl of an enzyme substrate solution (4 mg of orto-phenylenediamine, in 20 ml of 0.1 M phosphate-citrate buffer containing 0.001% H₂O₂) were added to each well and left in the dark for about 20 min. Enzymatic reaction was stopped with 50 µl of 4N H₂SO₄ and plates were read in a microplate autoreader at 450 nm. Results are expressed as the mean of the endpoint dilution of three sera per experimental condition, where the endpoint is determined as the final serum dilution that yields an absorbance value higher than the mean + 2 s.d. of three negative control samples included in each assay.

### DC preparation and injection

DC were isolated from spleens using a method previously described (28). Briefly, spleens from 129 or type I IFNR KO mice were cut into small pieces and digested, with agitation, in RPMI containing 5% FCS, Collagenase III (1 mg/ml, Lorne Laboratories, Reading, UK) and DNase I (0.6 mg/ml Sigma, St Louis, MO) for 5 min at 37°C followed by 15 min at RT. DC-enriched cell populations were obtained using Nycodenz (Life Technology Paisley, UK) gradients. The low-density cell fraction was then labeled with anti-CD11c-FITC (Becton Dickinson, Oxford, UK) in PBS-EDTA-FCS for 20 min on ice. After washing, the cells were filtered (70 µm cell strainer, Falcon) and CD11c⁺ cells were sorted on a MoFlow flow cytometer (Cytomation, Fort Collins, CO), with the resulting population being >98% CD11c⁺. After 2 washes in PBS, purified DC were incubated in PBS alone or in PBS containing 100 µg CGG for 30 min at 37° C. 5-7x 10⁵ purified DC, with or without CGG, were injected sc into type I IFNR KO mice ± 10⁵ U IFN-α/β. Mice receiving CGG + DC + IFN-α/β were given additional sc injections of 10⁵ U IFN-α/β 1 and 2 days later.

### T cell Proliferation and cytokine assays

*CGG-specific proliferation assays.*

DrLNs were cut into small pieces and digested in RPMI containing 5% FCS, collagenase III (1 mg/ml) and DNase I (0.6 mg/ml) for 20 min at RT with frequent mixing. Cell suspensions were then filtered (70 µm), washed and centrifuged at 1500 rpm for 10 min. For proliferation assays, unseparated cells (5 x 10⁵ per well) were cultured in complete medium (RPMI 1640 supplemented with 10% heat-inactivated FCS (PAA Laboratories), 50 µM 2-ME (Sigma) , 10 mM HEPES, 5% NCTC medium, 100 U/ml penicillin, 100 µg/ml streptomycin and 250 µg/ml gentamicin (all from Life Technologies)) in triplicate wells of 96-well plates ± CGG (20 µg/ well). On the 4th day of culture, wells were pulsed with 1 µCi [³H]-thymidine for 8 hours. Plates were then harvested and incorporated [³H]-thymidine measured using a MicroBeta TRILUX counter (Wallac, Turku, Finland). For cytokine assays, DrLN cells were incubated with anti-Class II (TIB120), anti-CD8 (3155) and anti-CD11b (M1/70) for 15 min on ice. After washing, CD4⁺ T cells were purified by negative selection using sheep anti-rat IgG and anti-mouse IgG magnetic Dynabeads (Dynal, Oslo, Norway). 2 X 10⁴ purified CD4⁺ T cells were cultured in complete medium in triplicate wells of 96-well plates with 5 X 10⁵ T-depleted splenocytes from non-treated syngeneic mice. T-depleted splenocytes were prepared by incubating spleen cells for 45 min at 37°C with rat anti-mouse-Thy-1 antibody (T24) and guinea pig complement (VH BIO Ltd, Gosford, UK). Before culture, T-depleted splenocytes were preincubated ± CGG (20 µg/well) for 1 hour at 37°C and irradiated at 3000 rads. After 3 days of culture, supernatants were harvested and cytokines measured using the Quantikine M kits for mouse IFN-γ and IL-4 from R&D (Abingdon, Oxon, UK) as directed by the manufacturer.

### OVA-specific proliferative assay

To test antigen-induced proliferative response, mice were sacrificed 32-35 days after the first immunization. Cells of a single-spleen cell suspension from each mouse spleen were cultured in a flat bottomed 96 well tray at a concentration of 5x10⁵ in 0.2 ml/well of 10%FCS RPMI medium containing different concentrations of OVA (0, 50, 100 and 200 µg/ml). After 4 days incubation at 37°C in 5% CO₂ humidified incubator, 0.5 µCi of ³H thymidine (DuPont-NEN, Boston, MA) were added. After further 18h incubation, cells were harvested on filtermate A (Wallac, Turku, Finland) and radioactivity was read on a scintillator (Betaplate, Wallac, Turku, Finland). Results are expressed as mean cpm ± SD. of three mice tested in triplicate.

### CGG-Specific Elispot Assay

Multi-Screen-IP sterile Elispot plates (Millipore, Walford, UK) were coated overnight with CGG at 20 µg/ml in Carbonate buffer pH 9.6. After 5 washes in PBS, plates were blocked for 2 hours with 4% milk in PBS at 37°C and washed 5 times in PBS. Cell suspensions were prepared from DrLNs as described above, washed, centrifuged at 1500 rpm for 10 min and resuspended in complete medium supplemented with 15 % FCS. All samples were plated in triplicate at several different cell concentrations (from 2 X 10⁴ - 5 X 10⁵ cells/well). Following overnight culture at 37°C in 5% CO₂, plates were then extensively washed with PBS-Tween (0.05%). CGG-specific antibodies were revealed by incubating the wells with isotype specific polyclonal goat anti-mouse antibodies conjugated to AP (Southern Biotechnology) for 2 hours at RT. After washes, BCIP (Sigma) diluted in (0.1M Tris/HCl, pH 9.5; 10% diethanolamine; 0.1M NaCl, 5mM MgCl₂) at 1mg/ml was used as the substrate for AP. The reaction was stopped by washing the plates with tap water. Spots were counted under a microscope.

### Example 1. Antibody response in mice immunized with CGG+ poly IC: importance of type I IFN

The ability of type I IFN to act as an adjuvant was first tested by using polyinosinic: polycytidylic acid (poly IC), a synthetic double stranded RNA, to induce production of type I IFN *in vivo.* C57BL/6 (B6) mice were immunized by injecting chicken gamma globulin (CGG) in PBS sc, and the effect of co-injecting poly IC examined. (C57BL/6 (B6) mice were immunized by injecting 100 µg of chicken gamma globulin (CGG) in PBS s.c., or the same amount of CGG mixed with 100 µg of poly IC in PBS sc). Ten days after immunization, the sera were assayed by ELISA for the presence of CGG-specific antibodies of various isotypes.

The relevant results are reported on figure 1A. CGG alone was poorly immunogenic, and the response was largely restricted to antibodies of the IgG1 subclass; IgM, IgG2b, IgG2a and IgG3 antibodies were detected at very low levels. Co-injection of poly IC stimulated a clear-cut increase in CGG-specific antibody titre, which applied to all subclasses of IgG (Figure 1A). This included 3-, 4.2-, 9- and 8.4-fold increases in the titres of IgG1, IgG2b, IgG2a and IgG3 antibodies respectively.

Although poly IC is known to be a potent inducer of type I IFN, it also induces other cytokines. Therefore, it was important to determine whether the adjuvant activity of poly IC was in fact dependent on type I IFN. To do so, we compared the ability of poly IC to enhance the antibody response in mice lacking a functional receptor for type I IFN (type I IFNR KO mice, which were on a 129 background) and in control (129) mice (immunisations were performed as described above).

As in B6 mice, poly IC markedly enhanced the antibody response to CGG in control 129 mice (Figure 1B). In contrast, poly IC had a greatly reduced ability to do so in type I IFNR KO mice. Small increases in IgM, IgG1 and IgG2b titres were observed in type I IFNR KO mice, indicating that poly IC can enhance the production of these isotypes independently of type I IFN. However, most of the effect of poly IC was dependent on type I IFN, since the titres of these antibodies remained much lower in type I IFNR KO mice than in control mice.

Furthermore, production of IgG2a and IgG3 anti-CGG antibodies was not stimulated at all by poly IC in type I IFNR KO mice. Taken together, these data show that induction of expression of type I IFN in the host stimulates a markedly increased antibody response to a soluble protein antigen, which includes antibodies of all IgG subclasses.

### Example 2. Antibody response in mice immunized with OVA+adjuvants: importance of type I IFN

Mice were treated with a first i.d. injection of OVA, OVA+IFA, OVA+ CFA, OVA+CpG or OVA+ alum. For the antigen a dose of 10 µg in 50 µl was used, IFA and CFA were mixed with antigen at a 1:1 v/v ratio and emulsified until a stable emulsion was obtained, CpG was used in the amount of 10 µg and mixed with antigen, and alum was used in a sufficient amount for adsorbing OVA. Following the first immunization, a second (day 10) and a third (day 17) treatment with OVA alone was performed. Control mice were treated with saline. The results showed that OVA alone was poorly immunogenic, eliciting a very low antibody response, while co-injection of the adjuvant induced an increase of OVA-specific antibody response (Fig. 2). This enhancement was particularly marked in the case of IFA, CFA, or CpG, and less pronounced for alum. IgG subclass characterisation showed that IFA and CFA acted as effective adjuvant for both IgG1 and IgG2a subclasses (typically associated with Th-2 and, respectively, Th-1 types of antibody response) (Fig. 2A), while CpG was more specific for IgG2a (Th-1 type) and alum for IgG1 (Th-2 type)(Fig. 2B). To determine whether the adjuvant activity is mediated by type I IFN, we compared the ability of all these adjuvants to enhance the antibody response in mice lacking a functional receptor for type I IFN (type I IFNR KO mice C3H/HeJ). The results showed that OVA-specific IgG2a and IgG1 subclasses were only slightly increased in type I IFNR KO mice with respect to control mice (Fig. 2A and 2B). This confirmed the functional role played by type I IFN in the enhancement of antibody response elicited by typical Th-1 promoting adjuvants.

### Example 3. In vitro proliferation and IFN gamma production of T cells from mice immunized with CGG+poly IC: importance of type I IFN

The effect of type I IFN on T cell priming was also assessed. This was done initially by measuring the capacity of LN T cells to proliferate upon re-stimulation with CGG in vitro. Draining LNs (DrLNs) were removed 10 days after immunization (as described in Example 1) and the resulting cell suspensions cultured in the presence or absence of CGG. Co-injection of poly IC with CGG into control (129) mice led to a much higher CGG-specific *in vitro* proliferative response than immunization with CGG alone (Figure 3A); pulsing with BrdU showed that most of the cells proliferating *in vitro* were CD4⁺ (data not shown). The enhancement of T cell priming was partially independent of type I IFN, since poly IC treatment of type I IFNR KO mice also resulted in some increase in the in vitro proliferative response (Figure 3A). However, the proliferation of cells from CGG + poly IC-injected type I IFNR KO mice was much lower than that of cells from CGG + poly IC-injected control mice, indicating that type I IFN were in fact strongly enhancing the T cell response *in vivo.* This was also evident when cytokine production by *in vitro* re-stimulated CD4⁺ T cells was examined (Figure 3B).

Thus, while CD4⁺ DrLN cells from mice immunized with CGG alone secreted little if any IFN-γ when stimulated by CGG *in vitro,* markedly higher amounts of IFN-γ were produced by CD4⁺ cells from poly IC + CGG-injected mice. Importantly, poly IC augmented the priming of IFN γ-secreting CD4⁺ T cells to a much greater extent in control mice than in type I IFNR KO mice. In contrast, low amounts of IL-4 were secreted from CD4⁺ cells in all groups that were not significantly different from each other. Thus, induction of type I IFN *in vivo* enhances T cell priming, promoting the generation of IFN γ-secreting CD4⁺ T cells.

### Example 4. Role of endogenous type I IFN in in vitro proliferation of T cells and in DTH response in mice immunized with OVA+adjuvants

At the end of the immunization described in Example 2, mice were sacrificed and spleens taken for a proliferation assay against OVA. In Fig. 4, the results of ³H thymidine uptake of splenocytes cultured with a medium containing 100 µg OVA are shown. In this experiment, all the spleen cells derived from type I IFN R^{+/+} mice immunized with OVA, OVA+CFA (Fig. 4A) or OVA, OVA+CpG, or OVA+alum (Fig. 4B) showed a significant proliferation (except for OVA+alum) with respect to saline-treated controls, whereas no proliferation was detected in any of the type I IFNR KO mouse treatment groups. In a parallel experiment, some normal and type I IFNR KO mice were immunized with slightly different protocol in which adjuvants were administered also in the second and the third immunization, and subsequently challenged with OVA into the footpad for DTH response measurement (Fig. 4C and 4D). The defective DTH response observed in type I IFNR KO mice as compared to controls indicates that the adjuvant-induced DTH response in mediated by endogenous type I IFN. In this regard, it is worth mentioning that all the adjuvants capable of inducing DTH also stimulate type I IFN production after injection in mice (not shown).

### Example 5. Stimulation of primary antibody responses by treatment with type I IFN

The effect of exogenous type I IFN on antibody responses was studied initially using a partially purified high titre preparation of murine type I IFN, which contained both IFN-α and IFN-β. B6 mice were injected sc with 100 µg of CGG alone or the same dose of CGG + 10⁵ U of IFN-α/β (IFN 1X). In addition, separate groups of mice injected with CGG + IFN-α/β received a second sc injection of IFN-α/β alone (10⁵ U) 1 day later (IFN 2X), or sc injections of IFN-α/β (10⁵ U) both 1 and 2 days later (IFN 3X). As shown in Fig. 5A, treatment of mice with IFN-α/β strongly enhanced the CGG-specific antibody response; the effect was most marked in mice receiving 3 injections of type I IFN and was apparent for all subclasses of IgG. CGG-specific IgE was not detectable in any group of mice (data not shown). Treatment with IFN-α/β similarly enhanced the antibody response in LPS-non-responsive C3H/HeJ mice, discounting the possibility of contamination with endotoxin (data not shown).

A similar experiment was performed using affinity-purified IFN-β (Figure 5B). In this case, a single injection of IFN-β was sufficient to enhance the primary antibody response, although, as for the partially purified IFN-α/β, the highest antibody titres were achieved after three injections of IFN-β. After one, two or three injections of IFN-β, antibody titres were increased respectively 5, 6 and 8-fold for IgM; 6.4, 8.5 and 12.8-fold for IgG1; 13.3, 16 and 26.6-fold for IgG2b; 25.6, 32 and 153.6-fold for IgG2a; 16.6, 64 and 117.3-fold for IgG3. Taken together with the experiment using partially purified IFN-α/β, these results clearly show that administration of type I IFN early during an immune response markedly increases the primary antibody response to a soluble protein antigen.

### Example 6. Antibody response in mice immunized with CGG + type I IFN pre-adsorbed to alum.

To determine whether prolonging the half-life of type I IFN would potentiate its ability to act as an adjuvant, type I IFN-α/βU) was mixed with CGG (100 µg) and a saturating amount of alum prior to sc injection; such a strategy has been shown to greatly augment the adjuvanticity of IL-12 (24). Strikingly, when pre-adsorbed to alum, a single injection of type I IFN enhanced the CGG-specific antibody response to a similar or greater extent than 3 injections of soluble type I IFN (Fig. 6).

The augmenting effect of alum pre-adsorption was most marked with regard to IgG2a production. This result suggests that prolonging the presence of type I IFN does indeed increase its adjuvant activity and may have practical implications regarding the use of type I IFN as an adjuvant.

### Example 7. Comparison between type I IFN and others adjuvants

To evaluate further the efficiency of type I IFN as an adjuvant, we compared their capacity to enhance the primary antibody response with that of commercial adjuvants. Initial comparisons were made with two oil-based adjuvants, Incomplete Freund's Adjuvant (IFA) and Titermax.

The adjuvants were mixed with antigen solution, containing 100 µg of CGG, at a 1:1 v/v ratio and emulsified until a stable emulsion was formed. Then, mice were immunized and sera analysed for the presence of anti CGG antibodies. Although IFA and Titermax stimulated higher levels of IgG1 antibodies, type I IFN were equivalent to these adjuvants in ability to induce IgM and IgG2b antibodies, and far superior in increasing the production of IgG2a and IgG3 antibodies (Fig. 7).

As a stricter test of adjuvant activity, type I IFN were compared to Complete Freund's Adjuvant (CFA). CFA has long been considered the "gold standard" for adjuvant activity in mice, and is known to enhance the production of antibodies of all isotypes. Thus, we compared CGG-specific antibody titres, 10 days after immunization, in control (WT 129) and IFN-IR KO mice injected with CGG alone, CGG + CFA or CGG + IFN-α/β (Fig. 8).

In control mice, antibody titres were higher in mice injected with CGG + IFN-α/β or CGG + CFA than in those immunized with CGG alone. Remarkably, the adjuvant activity of IFN-α/β compared favourably with that of CFA. In fact, although CFA induced higher titres of IgM antibodies (on the 129 background only), IFN-α/β stimulated the production of similar titres of IgG1 and IgG2b antibodies. Furthermore, IFN-α/β induced higher levels of IgG2a and, at least on the 129sv background, IgG3 antibodies than CFA. These results showed, therefore, that IFN-α/β does indeed have powerful adjuvant activity.

The effects are particularly significant when it is considered that the responses being compared were those to soluble protein + soluble IFN-α/β, which are likely cleared rapidly, and to the oily emulsion of CFA, which can persist at the site of injection for a long period of time.

### Example 8. Role of endogenous type I IFN in the adjuvant activity of CFA and in stimulation of the response to protein alone

A notable difference between the adjuvant activities of CFA and IFA or Titermax is that only the former was able to induce significant titres of IgG2a or IgG3 antibodies. Since this is a property shared by type I IFN, it raised the question of whether the ability of CFA to do so was related to induction of endogenous type I IFN by this adjuvant. That type I IFN were induced by CFA seemed likely, given that a key constituent of CFA is heat-killed mycobacteria, and bacterial components such CpG DNA are known to stimulate production of type I IFN.

To test this hypothesis, we compared the abilities of IFN-α/β and CFA to augment the antibody response to CGG in type I IFNR KO mice *vs* control mice (Fig. 8). As expected, IFN-α/β was completely unable to enhance the response to CGG in type I IFNR KO mice. Importantly, the ability of CFA to promote the antibody response was also highly deficient in type I IFNR KO mice. Although CFA still induced high titres of IgG1 antibodies in type I IFNR KO mice, there was no longer any enhancement of IgM, IgG2b, IgG2a or IgG3 antibodies compared to immunization with CGG alone. These results demonstrate an important role for type I IFN in the adjuvant activity of CFA.

### Example 9. Induction of long-term antibody production and memory by type I IFN

Having shown that type I IFN enhanced the primary antibody response, it was of interest to determine whether this response was long lasting. Initially, we tested for long-term antibody production by assaying the sera of mice 6 months after a single injection of CGG, or CGG + 3 injections of IFN-α/β as described in Example 5 (Fig. 9A). Mice primed with CGG alone had extremely low levels of CGG-specific antibody after 6 months.

In contrast, mice primed with CGG + IFN-α/β still had significant titres of CGG-specific antibodies in their sera. With the exception of IgG3, for which titres were very low and not significantly different from those in mice primed with CGG alone, antibodies of all tested isotypes were present. Thus, injection of type I IFN during priming allowed for long-term antibody production.

It remained controversial whether long-term antibody production is maintained by long-lived plasma cells or by replenishment of antibody-producing cells from memory B cells. Therefore, it was of interest to investigate whether memory was also induced by immunization in the presence of type I IFN. To do so, we examined the ability of mice primed 6 months earlier to mount a secondary response to CGG. Thus, mice who had been injected 6 months previously with CGG alone or CGG + 3 injections of IFN-α/β were re-injected with CGG alone (100 µg). To minimise the contribution from a primary response to CGG, the secondary response was studied on day 6 after challenge and naïve, non-primed mice were used as controls. CGG-specific antibody titres were compared in the same mice before and after re-injection of CGG (Fig. 9B).

In mice primed 6 months earlier with CGG alone, the response to CGG challenge was indistinguishable from that in naïve mice, indicating that there was no memory to CGG 6 months after priming with CGG alone. In marked contrast, however, mice primed 6 months previously with CGG + IFN-α/β did mount a rapid secondary response to CGG. The secondary response appeared, however, to be restricted to IgG2b and IgG2a antibody isotypes, despite the fact that high titres of IgG1 antibodies persisted in these mice. These results clearly showed that type I IFN promoted the generation of long-lived memory after a single injection of a soluble protein antigen.

### Example 10. Enhancement of the antibody response and isotype switching occurs through stimulation of dendritic cells by type I IFN

While type I IFN were clearly capable of markedly augmenting both the magnitude of the antibody response and switching to various IgG subclasses, their mechanism of action *in vivo* was unknown. We designed an adoptive transfer model in which DC were the only cells capable of responding to IFN-α/β In these experiments, 5-7 x 10⁵ highly purified splenic DC from wild type 129 mice or type I IFNR KO mice were incubated briefly with 100 µg of CGG and injected sc into type I IFNR KO recipients with or without IFN-α/β (10⁵ U) Mice receiving CGG + DC + IFN-α/β were given two further injections of IFN-α/β as before. CGG-specific antibody titres were then measured on day 10 after immunization (Figure 10).

As expected, IFN-α/β treatment of mice receiving CGG + type I IFNR KO DC did not enhance the antibody response, since no cells in these mice were able to respond to type I IFN. Conversely, injection of IFN-α/β into mice receiving CGG + wild type DC induced an increase in antibody titre for all four IgG subclasses compared to injection of CGG + wild type DC alone. Therefore, not only does stimulation of DC by type I IFN enhance the antibody response to co-injected protein, it is sufficient to induce isotype switching.

### Example 11. Antibody response of mice immunized with type IFN adjuvanted influenza vaccine.

We injected i.m. or i.n. C57BL/6 (B6) mice with 15 µg of purified sub-unit influenza vaccine alone or in association with 2x10⁵ U of type I IFN. Seven or fourteen days after immunization, the sera were assayed by ELISA for the presence of influenza-specific antibodies. Type I IFN co-injection resulted in a strong dose dependent adjuvant effect on influenza-specific antibody response (Fig. 11A). Fig. 11B shows that a prolonged type I IFN administration for two days after antigen injection further increased influenza-specific antibody response.

Notably, IFN-adjuvanted vaccine induced homogenous responses in all treated mice, whereas vaccine alone induced antibody response in a limited number of mice (about 30%) even after repeated immunizations. Fig. 11C shows the differential effects of using type I IFN as an adjuvant when administered at different times before, after or together with the vaccine. The optimal adjuvant effect was observed when IFN was co-injected with the vaccine. Fig. 12a shows that intranasal immunization with type I IFN-adjuvanted vaccine rendered the influenza vaccine highly immunogenic. Interestingly, the analysis of influenza specific antibody isotype showed the induction of IgG2a antibody subclass, typically associated with a Th-1 type immune response. Notably, IFN adjuvanted vaccine resulted in a stronger protective effect against virus challenge than vaccine alone (Fig. 12 B) .
1. Liu MA. Vaccine developments. Nat Med 4(5 Suppl):515-519, 1998.
2. Singh M and O'Hagan D. Advances in vaccine adjuvants. Nat Biotechnol 17:1075-1081, 1999.
3. Lindblad EB. The theory and practical application of adjuvants. In Stewart-Tull DES, ed., J. Wiley and Sons, Ltd, England 21-36, 1994.
4. Hilleman MR. Six decades of vaccine development - a personal history. Nat Med 4:507-514, 1998.
5. Lanzavecchia A and Sallusto F. From synapses to immunological memory: the role of sustained T cell stimulation. Curr Opin Immunol 12:92-98, 2000.
6. Kurosaki T. Functional dissection of BCR signaling pathways. Curr Opin Immunol 12:276-281, 2000.
7. Belardelli F. Role of interferons and other cytokines in the regulation of the immune response. APMIS 103:161-179, 1995.
8. Romagnani S. Induction of Th1 and Th2 responses: a key role for the «natural» immune response? Immunol Today 13:379-381, 1992.
9. Seder RA and Hill AVS. Vaccines against intracellular infections requiring cellular immunity. Nature, 406:793-798, 2000.
10. Belyakov I.M., Ahlers J.D., Clements J.D., Strober W. And Berzofsky J.A. Interplay of cytokines and adjuvants in the regulation of mucosal and systemic HIV-1-specific CTL. J Immunol 165:6454-6462, 2000.
11. Kim J.J., Simbiri KA, Sin JI, Dang K, Oh J, Dentchev T, Lee D., Nottingham LK, Chalian AA, McCallus D, Ciccarelli R, Agadjanyan MG, Weiner DB. Cytokine molecular adjuvants modulate immune responses induced by DNA vaccine constructs for HIV-1 and SIV. J Interferon Cytokine Res. 19:77-84, 1999.
12. Mogensen KE, Lewerenz M, Reboul J, Lutfalla G and Uze G. Type I IFN receptor: structure, function, and evolution of a family business. J Interferon Cytokine Res 19:1069-1098, 1999.
13. Belardelli F. and Gresser I. The neglected role of type I interferon in the T-cell response: implication for its clinical use. Immunol Today 17:369-372, 1996.
14. Gisler RH, Lindahl P and Gresser I. Effects of interferon on antibody synthesis in vitro. J Immunol 113:438-444, 1974.
15. Lindahl-Magnusson P, Leary P and Gresser I. Interferon inhibits DNA synthesis induced in mouse lymphocyte suspensions by phytohaemagglutinin or by allogeneic cells. Nat New Biol 237:120-121, 1972.
16. Zagury D, Lachgar A, Chams V, Fall LS, Bernard J, Zagury JF, Bizzini B, Gringeri A, Santagostino E, Rappaport J, Feldman M, Burny A and Gallo RC. Interferon alpha and Tat involvment in the immunosuppression of uninfected T cells and C-C chemokine decline in AIDS. Proc Natl Acad Sci USA 95:3851-3856, 1998.
17. Gringeri A, Santagostino E, Mannucci PM, Siracusano L, Marinoni A, Criscuolo M, Fall LS, M'Bika JP and Bizzini B. Anti-alpha interferon immunization: safety andimmunogenicity in asymptomatic HIV positive patients at high risk of disease progression. Cell Mol Biol 41:381-387, 1994.
18. Gringeri A, Santagostino E, Mannucci PM, Tradati F, Cultraro D, Buzzi A, Criscuolo M, David A, Guillemot L and Barre-Sinoussi. A randomized, placebo-controlled, blind anti-AIDS clinical trial: safety and immunogenicity of a specific anti-IFN alpha immunization. J Aquired Immun Defic Syndr 7:978
19. Tough DF, Borrow P, and Sprent J. Induction of bystander T cell proliferation by viruses and type I interferon *in vivo.* Science 272:1947-1950, 1996.
20. Glue P, Fang JW, Rouzier-Panis R, Raffanel C, Sabo R, Gupta SK, Salfi M, Jacobs S. Pegylated interferon-alpha2b: pharmacokinetics, pharmacodynamics, safety, and preliminary efficacy data. Hepatitis C Intervention Therapy Group. Clin Pharmacol Ther 68:556-672, 2000.
21. Putney SD and Burke PA. Improving protein therapeutics with sustained-release formulations. Nat Biotechnol 16:153-157, 1998.
22. Wang RF, Rosenberg SA. Human tumor antigens for cancer vaccine development. Immunol Rev 170:85-100, 1999.
23. Gilboa E. The makings of a tumor rejection antigen. Immunity 11:263-270, 1999.
24. Jankovic D, Caspar P, Zweig M, Garcia-Moll M, Showalter SD, Vogel FR and Sher A. Adsorption to aluminum hydroxide promotes the activity of IL-12 as an adjuvant for antibody as well as type 1 cytokine responses to HIV-1 gp120. J Immunol 159:2409-2417, 1997.
25. Tovey MG, Begon-Lours J and Gresser I. A method for the large scale production of potent interferon preparations. Proc Soc Exp Biol Med 146:809-815, 1974.
26. Belardelli F, Gessani S, Proietti E, Locardi C, Borghi P, Watanabe Y, Dawade Y, and Gresser I. Studies on the expression of spontaneous and induced interferons in mouse peritoneal macrophages by means of monoclonal antibodies to mouse interferons. J Gen Virol 68:2203-2212, 1987.
27. Kawade Y and Watanabe Y. Characterization of rat monoclonal antibodies to mouse interferon α and β. Proceedings of the third international TNO meeting on the biology of the Interferon system. In the Biology of the Interferon System, Dordrecht, 197-202, 1987.
28. Vremec D, Zorbas M, Scollay R, Saunders DJ, Ardavin CF, Wu L and Shortman K. The surface phenotype of dendritic cells purified from mouse thymus and spleen: investigation of the CD8 expression by a subpopulaton of dendritic cells. J Exp Med 176:47-58, 1992.

## Claims

1. Use of type I IFN for the preparation of vaccines including said type I IFN as an adjuvant in a dosage greater than or equal to 100.000 IU per dose of vaccine.

2. Use according to claim 1, wherein said type I IFN is natural IFN α, a synthetic recombinant type I IFN a recombinant IFN-α subtypes, IFN-β, IFN-ω, or a nucleic acid sequence encoding for one or more members of type I IFN.

3. Use according to claim 1 or 2, wherein said dosage is in the range of 1x10⁶ - 6x10⁶ IU.

4. Use according to any of claims 1 to 3, wherein said type I IFN is a pegylated type I IFN subtype.

5. Use according to any of claims 1 to 4, wherein said type I IFN is a recombinant type I IFNs fused with a monoclonal antibody capable of targeting dendritic cells.

6. Use according to claim 1 wherein said antigen is one or even more antigens from an infectious agent or other sources.

7. Use according to claim 1 wherein said antigen is one or even more antigens from tumors.

8. Use according to claim 6 or 7, wherein said antigen is in an amount of 1-1000 µg.

9. Use according to claim 8, wherein said amount is 10-200 µg.

10. Use according to any of claims 1 to 9, wherein said vaccine is formulated for delivering said antigen and said adjuvant simultaneously to the site of administration.

11. Vaccine comprising type I IFN as an adjuvant in a dosage greater than or equal to 100.000 IU per dose of vaccine.

12. Vaccine according to claim 11, wherein said type I IFN is type I IFN defined in claim 2, or in any of claims 3 to 5.

13. Vaccine according to claim 11 or 12, wherein said dosage is in the range of 1x10⁶-6x10⁶ IU.

14. Vaccine according to any of claims 11 to 13, wherein antigen is comprised in the amount defined in claim 8 or 9.

15. Vaccine according to any of claims 11 to 14, wherein aluminum salts are comprised in said vaccine.

16. Vaccine according to any of claims 11 to 15, wherein an additional dose of type I IFN is administered at day 1 or at day 1 and day 2 after vaccine injection.

17. Vaccine according to any of claims 11 to 15, wherein the formulation sustains controlled and prolonged release of both antigen and adjuvant.

18. Vaccine according to any of claims 11 to 17, formulated for delivering both antigen and adjuvant simultaneously to the site of administration.

19. A pharmaceutical composition comprising a vaccine as defined in any o claims 11 to 17 together with a pharmaceutically acceptable carrier vehicle or excipient therefor.

20. Kit of parts comprising
- a composition including type I IFN and pharmaceutically acceptable carrier vehicle or auxiliary agent; and
- a vaccine composition comprising an antigen and pharmaceutically acceptable carrier, vehicle or auxiliary agent;
for separate, simultaneous or sequential use in the prevention or treatment of a disease associated with the presence of said antigen.

21. Process for the preparation of the vaccine according to any of claims 11 to 18, comprising the step of:
- formulating an antigen together with type I IFN, said type I IFN being in a dosage greater than or equal to 100.000 IU per dose of vaccine.
